## Europäisches Patentamt
## European Patent Office
## Office européen des brevets

(19)

(11) Veröffentlichungsnummer: **0 129 900**
**B1**

(12) **EUROPÄISCHE PATENTSCHRIFT**

(45) Veröffentlichungstag der Patentschrift:
01.07.87

(21) Anmeldenummer: **84107278.8**

(22) Anmeldetag: **25.06.84**

(51) Int. Cl.⁴: **C 07 C 11/08**, C 07 C 5/23

(54) Verfahren zur Gewinnung von 1-Buten aus 2-Butene enthaltenden C4-Kohlenwasserstoffgemischen.

(30) Priorität: **25.06.83 DE 3323021**

(43) Veröffentlichungstag der Anmeldung:
**02.01.85 Patentblatt 85/1**

(45) Bekanntmachung des Hinweises auf die Patenterteilung:
**01.07.87 Patentblatt 87/27**

(84) Benannte Vertragsstaaten:
**AT BE DE FR GB IT NL**

(56) Entgegenhaltungen:
**FR-A-2 528 033**
**US-A-3 527 834**
**US-A-3 758 604**
**US-A-4 289 919**
**US-A-4 394 255**

(73) Patentinhaber: **BASF Aktiengesellschaft, Carl-Bosch- Strasse 38, D-6700 Ludwigshafen (DE)**

(72) Erfinder: **Lindner, Alfred, Dr., Ringstrasse 30, D-6712 Bobenheim- Roxheim (DE)**
Erfinder: **Wagner, Ulrich, Dr., Knospstrasse 7, D-6703 Limburgerhof (DE)**
Erfinder: **Volkamer, Klaus, Dr., Heidelberger Ring 21, D-6710 Frankenthal (DE)**
Erfinder: **Hoelderich, Wolfgang, Dr., Mannheimer Strasse 18 c, D-6710 Frankenthal (DE)**
Erfinder: **Hochstein, Waldhelm, Dr., Am Wurmberg 4, D-6713 Freinsheim (DE)**
Erfinder: **Immel Wolfgang, Dr., Carl- Bosch- Strasse 78, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft ein Verfahren zur Gewinnung von 1-Buten aus 2-Butene enthaltenden C4-Kohlenwasserstoffgemischen durch katalytische Isomerisierung der 2-Butene zu 1-Buten.

Es ist bereits aus der US-PS 2 921 103 bekannt, 2-Butene bei 610°C an einem Chromoxid-Aluminiumoxid-Katalysator unter Verwendung von Wasserdampf als Verdünnungsmittel zu 1-Buten zu isomerisieren. Bei diesem Verfahren werden jedoch trotz der Verwendung von Wasserdampf als Verdünnungsmittel nur Katalysatorlaufzeiten von 10 bis 100 Stunden erreicht. Wird das bekannte Verfahren ohne Zusatz von Wasserdampf durchgeführt, so werden gar nur Laufzeiten von bis zu 2 Stunden erhalten. Wegen der erforderlichen häufigen Katalysatorregenerierungen und wegen der erforderlichen hohen Wasserdampfverdünnung ist das Verfahren sehr arbeits- und energieaufwendig.

Weiter ist aus US-A-4 289 319 ein Verfahren zur Isomerisierung von 2-Buten an einem Manganoxid-Aluminiumoxid-Katalysator bekannt, bei dem gemäß dem Beispiel relativ hohe Reaktionstemperaturen und eine Verdünnung mit Stickstoff bei der Isomerisierung erforderlich sind. Eine Aufarbeitung des Isomerisierungsgemisches wird nicht beschrieben.

In US-A-3 758 604 wird schließlich ein Verfahren zur Abtrennung von Isobuten aus einem Isobuten und 1-Buten enthaltenden C4-Kohlenwasserstoffgemisch beschrieben bei dem das C4-Kohlenwasserstoffgemisch einer katalytischer Isomerisierung unterworfen wird, um das 1-Buten in die 2-Butene zu überführen und hierdurch eine leichtere destillative Abtrennung von Isobuten erreichen zu können.

Es wurde nun ein vorteilhaftes Verfahren gefunden zur Gewinnung von 1-Buten aus 2-Butene und gegebenenfalls 1-Buten enthaltenden C4-Kohlenwasserstoffgemischen durch katalytische Isomerisierung der 2-Butene zu 1-Buten bei erhöhten Temperaturen und Abtrennung des 1-Butens, welches dadurch gekennzeichnet ist, daß man die Isomerisierung der 2-Butene zu 1-Buten in Gegenwart eines sauren Katalysators bei Temperaturen von 200°C bis 450°C durchführt, das aus der Isomerisierungszone erhaltene Isomerisierungsgemisch in eine Destillationszone leitet, am Kopf der Destillationszone 1-Buten abzieht, im unteren Drittel der Destillationszone eine 2-Butene enthaltende Fraktion mit einem Gehalt von 1 bis 15 Gew.-% 1-Buten abzieht und in die Isomerinsierungszone leitet und das 2-Butene und gegebenenfalls 1-Buten enthaltende Ausgangs-C4-Kohlenwasserstoffgemisch der Destillationszone und/oder Isomerisierungszone zuführt.

Nach dem neuen Verfahren werden auch ohne Wasserdampfzusatz wesentlich längere Laufzeiten des Isomerisierungskatalysators erreicht als nach dem bekannten Verfahren. Das Verfahren zeichnet sich weiter, da ein Zusatz von Wasserdampf nicht erforderlich ist und die Isomerisierung bei relativ niedrigen Temperaturen durchgeführt wird, durch geringen apparativen Aufwand und Energieaufwand aus. Es war überraschend, daß sich das neue Verfahren in überaus wirtschaftlicher Weise durchführen läßt und sich der spezifische Energiebedarf für die 1-Butengewinnung niedrig halten läßt, da beim Übergang zu niedrigeren Temperaturen das Isomerisierungsgleichgewicht zwischen den 2-Butenen und 1-Buten zugunsten der 2-Butene verschoben wird. Es war weiterhin überraschend, daß auch ohne Wasserdampfzusatz die Bildung von Crackprodukten und Produkten der Skelettisomerisierung der Butene in der Isomerisierungsstufe so gering ist, daß auf weitere Reinigungsstufen zur Abtrennung von solchen Crackprodukten und Skelettisomerisierungsprodukten verzichtet werden kann und dabei 1-Buten in hoher Ausbeute erhalten werden kann.

Die für das erfindungsgemäße Verfahren einzusetzenden 2-Butene und gegebenenfalls 1-Buten enthaltenden C4-Kohlenwasserstoffgemische weisen im allgemeinen einen Gehalt an 2-Butenen und gegebenenfalls 1-Buten von 70 bis 100 Gew.%, in der Regel 80 bis 100 Gew.%, insbesondere 90 bis 100 Gew.% auf. 2-Buten kann als 2-Buten-cis oder 2-Buten-trans enthalten sein. In der Regel enthält das Ausgangs-C4-Kohlenwasserstoffgemisch jedoch ein Gemisch aus 2-Buten-cis und 2-Buten-trans. Neben den 2-Butenen kann das Ausgangs-C4-Kohlenwasserstoffgemisch bis zu 90 Gew.%, in der Regel zu 1 bis 70 % w.%, insbesondere zu 1 bis 60 Gew.% 1-Buten enthalten.

Die 2-Butene und gegebenenfalls 1-Buten enthaltenden C4-Kohlenwasserstoffgemische werden vorteilhaft durch extraktive Destillation unter Verwendung eines selektiven Lösungsmittels von Butane wie Isobutan und n-Butan und 2-Butene und gegebenenfalls 1-Buten enthaltenden C4-Kohlenwasserstoffgemischen erhalten, wobei zweckmäßig die im selektiven Lösungsmittel weniger löslichen Butane als Kopfprodukt der extraktiven Destillation abgetrennt werden, während gleichzeitig ein die Butene und das selektive Lösungsmittel enthaltender Extrakt aus der extraktiven Destillation abgezogen wird. Der erhaltene Extrakt wird anschließend vom selektiven Lösungsmittel befreit, wobei dann ein 2-Butene und gegebenenfalls 1-Buten enthaltendes C4-Kohlenwasserstoffgemisch erhalten wird, das für das erfindungsgemäße Verfahren als Ausgangs-C4-Kohlenwasserstoff verwendet werden kann. Als selektives Lösungsmittel für die Trennung der Butane und Butene kommen beispielsweise Carbonsäureamide, wie Dimethylformamid, Diethylformamid, Dimethylacetamid, N-Formylmorpholin, Acetonitril, Fufurol, N-Methylpyrrolidon, Butyrolacton, Aceton und ihre Mischungen mit Wasser in Betracht. Mit besonderem Vorteil wird N-Methylpyrrolidon als selektives Lösungsmittel verwendet.

Das 2-Butene und gegebenenfalls 1-Buten enthaltende Ausgangs-$C_4$-Kohlenwasserstoffgemisch kann für das erfindungsgemäße Verfahren der Destillationszone und bzw. oder der Isomerisierungszone zugeführt werden. Man wird das Ausgangs-$C_4$-Kohlenwasserstoffgemisch zweckmäßig der Destillationszone, z. B. im unteren oder vorzugsweise mittleren Drittel der Destillationszone, zuführen, wenn das Ausgangs-$C_4$-Kohlenwasserstoffgemisch neben den 2-Butenen bereits 1-Buten enthält, z. B. in einer Konzentration, die höher ist als die sich in der Isomerisierungszone einstellende 1-Buten-Konzentration. Dagegen wird man das Ausgangs-$C_4$-Kohlenwasserstoffgemisch, falls es einen hohen Gehalt an 2-Butenen, z. B. von mindestens 90 Gew.%, in der Regel mindestens 92 Gew.%, insbesondere mindestens 95 Gew.% aufweist, im allgemeinen am Sumpf der Destillationszone oder der Isomerisierungszone zuführen.

Als Katalysator für die Isomerisierung der 2-Butene zu 1-Buten werden saure Katalysatoren verwendet. Die sauren Katalysatoren können in flüssiger Phase, z. B. als wäßrige Mineralsäuren, vorzugsweise wäßrige Phosphorsäuren, z. B. Phosphorsäuren mit einem Gehalt an $P_2O_5$ von 1 bis 80 Gew.%, vorzugsweise 2 bis 75 Gew.%, oder als feste Katalysatoren verwendet werden.

Als feste saure Katalysatoren sind feste Phosphorsäurekatalysatoren, die Mono- oder vorzugsweise Polyphosphorsäure auf einem festen Trägermaterial enthalten, vorteilhaft geeignet. Geeignete Trägermaterialen für die Phosphorsäurekatalysatoren sind z. B. Aluminiumoxid, Kieselsäure, Aktivkohle, Kieselgur oder Bims. Vorzugsweise wird Kieselgel als Trägermaterial verwendet.

Weitere geeignete saure Katalysatoren sind saure Metallsulfate wie Natriumhydrogensulfat, Calciumhydrogensulfat, Aluminiumsulfate, Nickelsulfate, Kupfersulfat, Kobaltsulfat, Cadmiumsulfat, Strontiumsulfat. Diese sauren Metallsulfate können als solche verwendet werden. Vorzugsweise werden sie auf einem Trägermaterial angewendet. Geeignete Trägermaterialien sind z.B. Kieselgel, Aktivkohle, Aluminiumoxid oder Bims.

Weiter kommen Kieselgel oder Aluminiumoxid alleine sowie vorteilhaft Zeolithe als Katalysatoren für die Zerlegung in Betracht. Mit besonderem Vorteil werden Boro- oder Aluminosilikatzeolithe vom Pentasiltyp verwendet.

Zur Erhöhung der Selektivität, der Standzeit und der Anzahl der möglichen Regenerierungen lassen sich unterschiedliche Modifizierungen an den zeolithischen Katalysatoren vornehmen.

Eine Art der Modifizierung besteht darin, daß man den unverformten oder verformten Zeolithen mit Alkalimetallen wie Na, Erdalkalimetallen wie Ca, Mg, Erdmetallen wie B, Tl, Übergangsmetallen wie Mn, Fe, Mo, Cu, Zn und/oder seltenen Erdmetallen wie La, Ce, ionenaustauscht oder dotiert. Eine Ausführungsform für diese Modifizierung besteht beispielsweise darin, daß man den verformten Pentasilzeolithen in einem Steigrohr vorlegt und bei Raumtemperatur oder erhöhten Temperaturen, z. B. bei Temperaturen von 20° C bis 120° C, vorzugsweise 20° C bis 100° C, ein Salz der voranbeschriebenen Metalle, z. B. ein Halogenid oder ein Nitrat, darüberleitet. Ein derartiger Ionenaustausch kann z. B. an der Wasserstoff-, Ammonium- oder Alkaliform des Zeolithen vorgenommen werden. Eine weitere Ausführungsform für die Modifizierung besteht darin, daß man das zeolithische Material mit einer Verbindung der voranbeschriebenen Metalle, z. B. mit einem Halogenid, Nitrat und/oder Oxid, in flüssigem Medium, z. B. in wäßriger oder alkoholischer Lösung, imprägniert. Sowohl an einen Ionenaustausch als auch an eine Imprägnierung schließt sich zweckmäßig zumindest eine Trocknung und/oder Calcination an. Es kann vorteilhaft sein, bei den metalldotierten Zeolithen eine Nachbehandlung mit Wasserstoff anzuschließen.

Eine weitere Möglichkeit der Modifizierung besteht darin, daß man das unverformte oder verformte zeolithische Material einer Behandlung mit Säuren wie Salzsäure, Flußsäure, Phosphorsäure unterwirft. Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z. B. durch Abfiltrieren und Auswaschen, des zeolithischen Materials wird dieses zweckmäßig, z. B. bei Temperaturen von 100° C bis 160° C, getrocknet und bei Temperaturen von im allgemeinen 450° C bis 600° C calciniert. Gemäß einer weiteren bevorzugten Ausführungsform für die Säurebehandlung wird das zeolithische Material nach seiner Verformung mit Bindemittel bei erhöhter Temperatur, zweckmäßig bei Temperaturen von 50° C bis 90° C, vorzugsweise 60° C bis 80° C, über einen Zeitraum von 0,5 bis 5, vorzugsweise 1 bis 3 Stunden mit Salzsäure, im allgemeinen mit 3 bis 25 gew.%iger Salzsäure, vorzugsweise mit 12 bis 20 gew.%iger Salzsäure, behandelt. Anschließend wird das zeolithische Material im allgemeinen ausgewaschen und zweckmäßig, z. B. bei Temperaturen von 100° C bis 160° C, getrocknet und bei Temperaturen von im allgemeinen 450° C bis 600° C calciniert.

In einer weiteren Ausführungsform des erfindungsgemäßen Verfahrens wird für die Isomerisierung als saurer Katalysator ein Metallphosphat, insbesondere ein Metallhydrogenphosphat, verwendet. Diese Phosphate können Phosphorsäure auch im Überschuß, der über die stöchiometrische Zusammensetzung der sauren Metallphosphate hinausgeht, enthalten, z. B. in einem Überschuß bis zu 65 %, vorzugsweise bis zu 20 %, insbesondere bis zu 10 %. Als derartige Metallphosphate können beispielsweise Magnesiumphosphat, Calciumphosphate, Strontiumphosphate, Bariumphosphate, Manganphosphate, Nickelphosphate, Kupferphosphate, Kobaltphosphate, Cadmiumphosphate, Eisen(II)-phosphate, Chromphosphate und insbesondere Aluminiumphosphate verwendet werden. Der Metallphosphat-Katalysator kann als solcher oder auf einem Trägermaterial verwendet werden. Geeignete Trägermaterialien sind beispielsweise Aluminiumoxid, Kieselsäure, Aktivkohle, Zinkoxid.

Die Menge des sauren Katalysators beträgt im allgemeinen 0,01 bis 10 kg, vorzugsweise 0,03 bis 2 kg je kg/h Durchsatz der zu isomerisierenden 2-Butene durch den Reaktor. Vorzugsweise werden für die Isomerisierung Festbettreaktoren verwendet.

Bei Nachlassen der Aktivität der festen sauren Katalysatoren werden diese zweckmäßig regeneriert. Eine vorteilhafte Methode zur Regenerierung der festen Katalysatoren besteht darin, daß man den Katalysator mit Phosphorsäure, vorzugsweise mit wäßriger Phosphorsäure behandelt. Diese Behandlung mit Phosphorsäure kann kontinuierlich, d.h. ohne Unterbrechung der Isomerisierungsreaktion durchgeführt werden, indem man zweckmäßig im Bereich der Isomerisierungstemperatur während der Isomerisierung zweckmäßig wäßrige Phosphorsäure auf den Katalysator aufgibt, wobei die Phosphorsäure im allgemeinen in einer solchen Menge zugegeben wird, daß, bezogen auf den Katalysator und berechnet als $P_2O_5$, stündlich $10^{-6}$t bis $10^{-3}$, vorzugsweise $10^{-5}$ bis $10^{-4}$ kg $P_2O_5$ je kg Katalysator zugegeben werden. Die Regenerierung des festen Katalysators kann auch diskontinuierlich, d.h. unter Unterbrechung der Isomerisierungsreaktion, durchgeführt werden. Die diskontinuierliche Isomerisierung erfolgt ebenfalls zweckmäßig bei erhöhter Temperatur, z. B. bei Temperaturen von 50 bis 300° C, vorzugsweise 50 bis 250° C, insbesondere 50 bis 150° C, wobei die Phosphorsäure im allgemeinen in einer solchen Menge auf den Katalysator aufgegeben wird, daß, bezogen auf den Katalysator und berechnet als $P_2O_5$, etwa 0,1 bis 20 Gew.% $P_2O_5$ zugegeben werden. Im allgemeinen erfolgt die Behandlung des festen Katalysators mit Phosphorsäure in der Weise, daß man die Phosphorsäure zweckmäßig als wäßrige Phosphorsäure über den Katalysator rieseln läßt.

Der feste saure Katalysator läßt sich weiter vorteilhaft durch Abbrennen mit Sauerstoff enthaltenden Gasen, vorzugsweise Luft, regenerieren. Es kann vorteilhaft sein, die Luft mit inerten Gasen, z. B. Stickstoff zu verdünnen. Die Behandlung der Katalysatoren mit den Sauerstoff enthaltenden Gasen wird im allgemeinen bei Temperaturen von 300° C bis 500° C durchgeführt.

Die Isomerisierung der 2-Butene zu 1-Buten in der Isomerisierungszone wird bei Temperaturen vor 200° C bis 450° C, vorzugsweise 250° C bis 400° C durchgeführt. Die Isomerisierung kann bei Atmosphärendruck durchgeführt werden. Vorzugsweise erfolgt die Isomerisierung jedoch bei erhöhtem Druckzweckmäßig bei Drücken bis zu 100 bar, wobei die Verwendung fester Katalysatoren vorzugsweise Drucke von 2 bis 60 bar, insbesondere 6 bis 40 bar, und bei Anwendung flüssiger Katalysatoren, vorzugsweise Drucke von 10 bis 100 bar, insbesondere 30 bis 50 bar, angewendet werden.

Es ist ein Vorteil des erfindungsgemäßen Verfahrens, daß ein Wasserdampfzusatz zu dem in die Isomerisierungszone eingeleiteten die 2-Butene enthaltenden $C_4$-Kohlenwasserstoffgemisch nicht erforderlich ist. Es ist zwar auch möglich, das zu isomerisierende C4-Kohlenwasserstoffgemisch mit Wasserdampf zu verdünnen. Dabei wird man jedoch den Wasserdampfzusatz zu dem zu Isomerisierenden $C_4$-Kohlenwasserstoffgemisch in der Regel auf weniger als 1 Mol, vorzugsweise auf höchstens 0,8 Mol, insbesondere auf höchstens 0,5 Mol Wasser je Mol 2-Butene begrenzen.

Das aus der Isomerisierungszone erhaltene Isomerisierungsgemisch, wird anschließend zur Abtrennung des 1-Butens von den 2-Butenen in eine Destillationszone geleitet, in der das 1-Buten zweckmäßig durch konventionelle Destillation als Kopfprodukt abgezogen wird. Dabei werden für die Destillation im allgemeinen übliche Destillationskolonnen mit beispielsweise ca. 100 praktischen Böden verwendet.

Im unteten Drittel der Destillationsionszone wird eine die 2-Butene enthaltende Fraktion, die einen gehalt von 1 bis 15 Gew.-%, vorzugsweise 1 bis 12 Gew.-%, insbesondere 2 bis 10 Gew.-& 1-Buten aufweist, abgezogen und in die Isomerisierungszone zurückgeführt. Überraschenderweise wird bei Einstellen dieses relativ hohen Gehaltes an 1-Buten in der aus dem unteren Drittel der Destillationszone in die Isomerisierungszone zuruckgeführten Fraktion der spezifischen Energiebedarf beim erfindungsgemäßen Verfahren abgesenkt, zumal durch diese Ruckführung von 1-Buten in die Isomerisierungszone der Abstand der 1-Buten-Konzentration in der zurückgeführten Buten-Fraktion von der dem Isomerisierungsgleichgewicht in der Isomerisierungszone entsprechenden 1-Buten-Konzentration verringert wird.

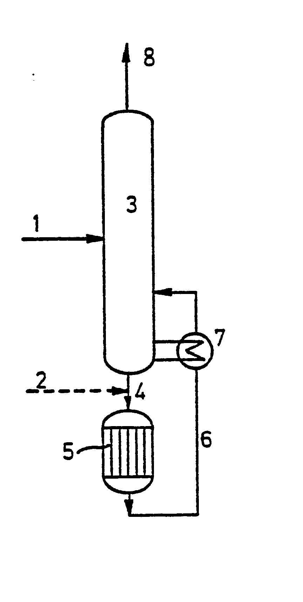

In der Figur wird eine beispielhafte Ausführungsform des erfindungsgemäßen Verfahrens schematisch erläutert. Das 2-Butene und gegebenenfalls 1-Buten enthaltende Ausgangs-$C_4$-Kohlenwasserstoffgemisch wird durch Leitung 1 der Destillationskolonne 3 zugeführt und bzw. oder durch Leitung 2 und 4 direkt dem Isomerisierungsreaktor 5 zugeführt.

Am Boden der Destillationskolonne wird eine Butene enthaltende Sumpffraktion abgezogen und über Leitung 4 dem Isomerisierungsreaktor zugeführt, in dem an einem sauren Katalysator die Isomerisierung der 2-Butene zu 1-Buten erfolgt. Aus dem Isomerisierungsreaktor wird das Isomerisierungsgemisch über Leitung 6 abgezogen und der Destillationskolonne zweckmäßig in der unteren Hälfte der Destillationskolonne zugeführt, wobei zweckmäßig die fühlbare Wärme des Isomerisierungsgemischs im Aufkocher 7 zur Aufkochung der Destillation verwendet wird. Es kann jedoch auch ein Wärmetausch mit der über Leitung 4 dem Isomerisierungsreaktor zugeführten Butene enthaltenden Fraktion erfolgen. Am Kopf der Destillationskolonne wird über Leitung 8 1-Buten abgezogen, das eine hohe Reinheit aufweist und in der Regel ohne weitere Reinigungsoperationen weiterverarbeitet werden kann.

1-Buten ist ein wichtiger Ausgangsstoff beispielsweise für die Herstellung von Polymeren wie Polybuten-1 oder Copolymeren, z. B. mit Ethen zur Herstellung von linearem Low density Polyethylen (LDPE), sowie zur Herstellung von Butenoxid.

Das folgende Beispiel veranschaulicht die Erfindung.

### Beispiel

Für die Gewinnung von 1-Buten wurde ein Ausgangs-$C_4$-Kohlenwasserstoffgemisch der folgenden Zusammensetzung verwendet:

| | |
|---|---|
| 2-Buten-trans | 24,4 Gew.% |
| 2-Buten-cis | 19,8 Gew.% |
| 1-Buten | 55,8 Gew.% |
| Butane | Spuren |

Dieses Butene-Gemisch war erhalten worden, indem zunächst aus einer aus einer Ethylenanlage erhaltenen $C_4$-Fraktion durch extraktive Destillation das Butadien extrahiert worden war, in einer weiteren Stufe Isobuten unter Gewinnung von Methyl-tert.-butylether abgetrennt worden war und schließlich durch eine weitere extraktive Destillation die Butane abgetrennt worden waren.

100 g/h Butene-Gemisch der oben angegebenen Zusammensetzung wurden in das mittlere Drittel einer Destillationskolonne eingeleitet. Am Boden der Destillationskolonne wurden 825 g/h einer Sumpffraktion aus 95 Gew.% 2-Butenen und 5 Gew.% 1-Buten abgezogen und in einen Isomerisierungsreaktor geleitet, der einen Phosphorsäure-Kieselgel-Katalysator als Festbettkatalysator enthielt und in dem eine Reaktionstemperatur von 250°C bis 300°C aufrecht erhalten wurde. Das aus dem Isomerisierungsreaktor abgezogene Isomerisierungsgemisch aus 90 Gew.% 2-Butenen und 10 Gew.% 1-Buten wurde in das untere Drittel der Destillationskolonne geleitet. Am Kopf der Destillationskolonne wurden 100 g/h 1-Buten abgezogen mit einer Reinheit von 99,5 Gew.%. Die Ausbeute an 1-Buten betrug 178 %, bezogen auf das im Ausgangs-$C_4$-Kohlenwasserstoffgemisch enthaltene 1-Buten.

Bei der Isomerisierung aus dem eingesetzten Gemisch entstandene geringe Spuren an $C_8$-Kohlenwasserstoffen sowie geringe Mengen Butan konnten durch Rückführung eines Teils der Sumpffraktion der Destillationskolonne zur vorgeschalteten extraktiven Destillation ausgeschleust werden.

Durch die Kreisfahrweise konnten die n-Butene praktisch vollständig als 1-Buten erhalten werden.

Bei Absinken des Isomerisierungsumsatzes wurden folgende Maßnahmen durchgeführt:

1. Dem dem Isomerisierungsreaktor zugeführten Gemisch aus 2-Butenen und 1-Buten wurde Wasserdampf zugesetzt.

2. Der Isomerisierungskatalysator wurde zur Regenerierung mit wäßriger Phosphorsäure behandelt.

3. Der Isomerisierungskatalysator wurde zur Regenerierung bei 400°C mit einem Gemisch aus Luft und Stickstoff behandelt.

Während bei dem Wasserdampfzusatz (Maßnahme 1) kein Einfluß feststellbar war, konnte bei Regenerierung des Isomerisierungskatalysators gemäß den Maßnahmen 2 oder 3 der dem ursprünglichen Umsatz am frischen Katalysator entsprechende Umsatz wieder hergestellt werden.

### Patentansprüche

1. Verfahren zur Gewinnung von 1-Buten aus 2-Butene und gegebenenfalls 1-Buten enthaltenden $C_4$-Kohlenwasserstoffgemischen durch katalytische Isomerisierung der 2-Butene zu 1-Buten bei erhöhten Temperaturen und Abtrennung des 1-Butens, dadurch gekennzeichnet, daß man die Isomerisierung der 2-Butene zu 1-Buten in Gegenwart eines sauren Katalysators bei Temperaturen von 200°C bis 450°C durchführt, das aus der Isomerisierungszone erhaltene Isomerisierungsgemisch in eine Destillationszone leitet, am Kopf der Destillationszone 1-Buten abzieht, im unteren Drittel der Destillationszone eine 2-Butene enthaltende Fraktion mit einem Gehalt von 1 bis 15 Gew.-% 1-Buten abzieht und in die Isomerisierungszone leitet und das 2-Butene und gegebenenfalls 1-Buten enthaltende Ausgangs-C4-Kohlenwasserstoffgemisch der Destillationszone und/oder Isomerisierungszone zufuhrt.

2. Verfahren nach Anspruch 1, dadurch gekennzeichnet, daß das 2-Butene und gegebenenfalls 1-Buten enthaltende Ausgangs-C4-Kohlenwasserstoffgemisch durch extraktive Destillation mit Hilfe eines selektiven Lösungsmittels eines Butane und 2-Butene und gegebenenfalls 1-Buten enthaltenden C4-Kohlenwasserstoffgemischs erhalten wird.

3. Verfahren nach Anspruch 1 und 2, <u>dadurch gekennzeichnet</u>, daß man einen festen Katalysator verwendet und den festen Katalysator durch Behandlung mit Phosphorsäure regeneriert.

4. Verfahren nach Anspruch 1 und 2, <u>dadurch gekennzeichnet</u>, daß man einen festen Katalysator verwendet und den festen Katalysator durch Abbrennen mit Sauerstoff enthaltenden Gasen regeneriert.

**Claims**

1. A process for recovering 1-butene from a $C_4$-hydrocarbon mixture which contains 2-butenes and may or may not contain 1-butene by catalytic isomerization of the 2-butenes to 1-butene at elevated temperatures, and isolation of the 1-butene, wherein the isomerization of the 2-butenes to 1-butene is carried out in the presence of an acidic catalyst at from 200 to 450°C, the isomerization mixture obtained from the isomerization zone is fed into a distillation zone, 1-butene is taken off at the top of the distillation zone, a fraction containing 2-butenes and from 1 to 15 % by weight of 1-butene is taken off from the lower third of the distillation zone and fed into the isomerization zone, and the starting $C_4$-hydrocarbon mixture which contains 2-butenes and may or may not contain 1-butene is fed to the distillation zone and/or the isomerization zone.

2. A process as claimed in claim 1, wherein the starting $C_4$-hydrocarbon mixture which contains 2-butenes and may or may not contain 1-butene is obtained by extractive distillation, with the aid of a selective solvent, of a $C_4$-hydrocarbon mixture which contains butanes and 2-butenes and may or may not contain 1-butene.

3. A process as claimed in claims 1 and 2, wherein a solid catalyst is used, and the solid catalyst is regenerated by treatment with phosphoric acid.

4. A process as claimed in claims 1 and 2, wherein a solid catalyst is used, and the solid catalyst is regenerated by combustion with an oxygen-containing gas.

**Revendications**

1. Procédé de récuperation de 1-butène à partir de mélanges d'hydrocarbures en $C_4$ contenant des 2-butènes et éventuellement du 1-butène, par isomérisation catalytique des 2-butènes en 1-butène à températures elevées et par séparation du 1-butène, <u>caractérisé en ce que</u> l'on effectue l'isomérisation des 2-butènes en 1-butène en présence d'un catalyseur acide, à des températures de 200°C à 450°C, en ce que l'on amène le mélange d'isomérisation sortant de la zone d'isomérisation dans une zone de distillation, en ce que l'on separe en tête de la colonne de distillation du 1-butène, en ce que l'on sépare dans le tiers inférieur de la zone de distillation une fraction contenant des 2-butènes et 1 à 15 % en poids de 1-butène, fraction que l'on conduit vers la zone d'isomérisation, et en ce que l'on introduit le mélange d'hydrocarbures en $C_4$ de départ contenant des 2-butènes et éventuellement du 1-butène dans la zone de distillation et/ou dans la zone d'isomérisation.

2. Procédé suivant la revendication 1, caractérisé en ce que l'on obtient le mélange d'hydrocarbures en $C_4$ de départ contenant des 2-butènes et eventuellement du 1-butène par distillation extractive, au moyen d'un solvant sélectif, d'un mélange d'hydrocarbures en $C_4$ contenant des butanes et des 2-butènes et éventuellement du 1-butène.

3. Procédé suivant la revendication 1 ou 2, caractérisé en ce que l'on emploie un catalyseur solide et en ce que l'on régénère le catalyseur solide par traitement par l'acide phosphorique.

4. Procédé suivant la revendication 1 ou 2, caractérisé en ce que l'on utilise un catalyseur solide et en ce que l'on régénère le catalyseur solide par un brûlage au moyen de gaz contenant de l'oxygène.